(19) Europäisches Patentamt
European Patent Office
Office européen des brevets

(11) Publication number: 0 102 690
B1

# (12) EUROPEAN PATENT SPECIFICATION

(45) Date of publication of patent specification: 08.04.87

(51) Int. Cl.$^4$: **D 06 M 13/46**, D 06 M 13/34, D 06 M 13/40, C 07 C 143/74, C 07 C 121/43, C 07 D 303/36

(21) Application number: 83303341.8

(22) Date of filing: 09.06.83

## (54) Treatment of fibrous substrates, such as carpet with fluorochemical.

(30) Priority: 11.06.82 US 387444

(43) Date of publication of application: 14.03.84 Bulletin 84/11

(45) Publication of the grant of the patent: 08.04.87 Bulletin 87/15

(84) Designated Contracting States: BE DE FR GB IT

(56) References cited:
DE-A-1 793 486
DE-A-1 928 985
DE-A-2 016 019
FR-A-2 196 416

(73) Proprietor: MINNESOTA MINING AND MANUFACTURING COMPANY
3M Center, P.O. Box 33427
St. Paul, Minnesota 55133-3427 (US)

(72) Inventor: Patel, Kalyanji U.
MINNESOTA MINING AND MANUFACT. COMPANY 2501 Hudson Road P.O. Box 33427
Saint Paul Minnesota 55133 (US)

(74) Representative: Baillie, Iain Cameron et al
c/o Ladas & Parry Isartorplatz 5
D-8000 München 2 (DE)

EP 0 102 690 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the treatment of fibrous substrates, such as textiles, paper, and leather, and particularly to the treatment of carpet, with fluorochemical, and to the resulting treated substrates. In another aspect, it relates to fluorochemicals, and their preparation, which are useful in such treatment.

In the industrial production of textiles, such as carpet and apparel, and such other fibrous, porous substrates as paper and leather, it is common to treat the surfaces of such substrates with fluorochemicals containing fluoroaliphatic radicals (often designated by the symbol "$R_f$") to modify the surface properties of such substrates. The $R_f$ radicals have extremely low free-surface energy, high chemical stability, and are hydrophobic and oleophobic, and thus such chemicals impart oil and water repellency to the treated surface. Fluorochemicals of this type and their application to fibrous substrates are described in various prior art publications, e.g., U.S. Patent Nos. 3,329,661 (Smith et al), 3,458,571 (Tokoli), 3,574,791 (Sherman et al), 3,728,151 (Sherman et al), 3,916,053 (Sherman et al), 4,144,367 (Landucci), 4,165,338 (Katsushima et al), 4,215,205 (Landucci), 4,013,627 (Temple), and 4,264,484 (Patel) and GB—A—2 075 514.

DE—A—2,016,019 discloses fluorinated halomethyl ethers and quaternary salts thereof. DE—A—1,793,486 discloses fluoroalkylamines and fluoroalkylamide derivatives and their quaternary derivatives. Brace, N.O., "Cyclization of N-Substituted Diallylamines to Pyrrolidine Derivatives During the Radical Addition of Perfluoroalkyl Iodides", J. Org. Chem., Vol. 36, No. 21, 1971, pp. 3187—3191 discloses fluorochemical iodide compounds which form polymeric quaternary salts.

Although some fluorochemicals are useful in many applications and many are commercial products, some are relatively expensive to prepare and apply and others do not impart the required properties to the degrees desired.

Briefly, this invention provides in one aspect non-polymeric fluorochemicals, namely fluorochemical amine salt compounds, containing one or two fluoroaliphatic radicals ($R_f$) and one or two tertiary amine salt groups or quaternary amine salt groups which have as N-substituents groups electron-withdrawing cyanoalkyl moieties, such fluorochemicals being useful as treating agents, e.g. in the form of an aqueous dispersion of said salt, for fibrous substrates, such as textiles, e.g. carpet such as nylon carpet, to modify surface properties thereof, e.g. to impart oil and water repellency to the surface of said substrates. The treating agents of this invention are thus non-ionic compounds, i.e., salts of free amine compounds.

A class of such fluorochemical compounds can be represented by the general formula

I $$\left[ R_f-Q-\overset{R^1}{\underset{R^3}{N}}-R^2 \right]_y (R^4)_p \left[ A \right]_z$$

where

$R_f$ is a fluoroaliphatic radical,

Q is an organic linking moiety,

N is a tertiary amine nitrogen or a quaternary amine nitrogen,

$R^1$ is a hydrogen or an alkyl group,

$R^2$ is a monovalent group selected from the class of $R_fQ$—, alkyl and aryl groups, and combinations of such groups, e.g. alkaryl, which groups can contain a hetero atom, e.g. —O— or —S—, —RCONH— (where R is alkyl or aryl), and

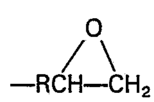

(where R is alkylene), or

$R^2$ is a divalent group selected from the class of alkylene and arylene groups, combinations thereof, and —RCONH— (where R is alkylene or arylene),

$R^3$ is an electron-withdrawing cyanoalkyl moiety,

$R^4$ is an alkylene or arylene group,

A is an anion derived from a protonic acid or an alkylating agent,

y is 1 or 2,

p is 0 or 1, and

z is 1 or 2,

with the provisos that

(1) when y is 1, then p is 0 and z is 1,

(2) when y is 2, then $R^2$ is said divalent group.

In the above formula, the alkyl groups in $R^1$, $R^3$, and R can have 1 to 6 carbon atoms, the alkyl in $R^2$ can have 1 to 18 carbon atoms, the alkylene groups of $R^2$ and $R^4$ can have 1 to 12 carbon atoms, and the aryl and arylene groups can have 6 to 12 carbon atoms.

A preferred subclass of the fluorochemicals of formula I is:

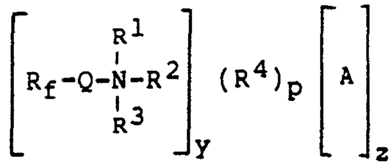 II

where

$R_f$, Q, $R^1$, $R^3$, $R^4$, A, y, p, and z are as defined for formula I,

$R^2$ is alkyl or alkylene as defined for formula I.

The fluoroaliphatic radical, $R_f$, is a fluorinated, stable, inert, non-polar, preferably saturated, monovalent moiety which is both oleophobic and hydrophobic. It can be straight chain, branched chain, and, if sufficiently large, cyclic, or combinations thereof, such as alkylcycloaliphatic radicals. The skeletal chain can include catenary oxygen and/or trivalent nitrogen hetero atoms bonded only to carbon atoms, such hetero atoms providing stable linkages between fluorocarbon groups and not interfering with the inert character of the $R_f$ radical. While $R_f$ can have a large number of carbon atoms, compounds where $R_f$ is not more than 20 carbon atoms will be adequate and preferred since large radicals usually represent a less efficient utilization of fluorine than is possible with smaller $R_f$ radicals. Generally, $R_f$ will have 3 to 20 carbon atoms, preferably 6 to about 12, and will contain 40—78 weight percent, preferably 50—78 weight percent, of fluorine. The terminal portion of the $R_f$ group has preferably at least three fully fluorinated carbon atoms, e.g. $CF_3CF_2CF_2$—, and the preferred compounds are those in which the $R_f$ group is fully or substantially completely fluorinated, as in the case where $R_f$ is perfluoroalkyl, $C_nF_{2n+1}$.

Generally, the fluorochemical compound will contain about 20 to 65 weight percent, preferably about 30 to 55 weight percent, of carbon-bonded fluorine. If the fluorine content is less than about 20 weight percent, impractically large amounts of the fluorochemical compound will generally be required, while fluorine contents greater than about 65 weight percent are unnecessary to achieve the desired surface properties and thus represent an uneconomical use of fluorine.

The function of the linkage or bridge Q is to bond the fluoroaliphatic radical, $R_f$, to the amine moiety of the compound. Q can comprise one or a combination of groups such as polyvalent aliphatic, e.g., $-CH_2-$, $-CH_2CH_2-$, and $-CH_2CH(CH_2-)_2$, polyvalent aromatic, oxy, thio, carbonyl, sulfone, sulfoxy, $-N(C_2H_5)-$, sulfonamido, carbonamido, sulfonamidoalkylene, carbonamidoalkylene, hydroxymethylene, carbonyloxy, urethane, e.g., $-CH_2CH_2OCONH-$, and urea, e.g., $-NHCONH-$. The linkage Q for a specific compound useful in this invention will be dictated by the ease of preparation of such a compound and the availability of necessary precursors thereof. From the above description of Q, it is apparent that this linkage can have a wide variety of structures. Since N is a nitrogen atom of a salt-forming amino group, it follows that the moiety of Q which is bonded to N cannot be an electrophilic moiety, such as carbonyl, sulfonyl, etc., which would preclude said nitrogen atom from being that of a salt-forming amino group. However large Q is, the fluorine content (the locus of which is $R_f$) of the compound is in the aforementioned limits.

The above-described fluorochemical compounds can be prepared by reacting fluoroaliphatic radical-containing intermediates (that is, $R_f$ intermediates, which generally are commercially made by electrochemical fluorination of organic acids or halides thereof or by telomerization of tetrafluoroethylene, followed by known reactions to form said intermediates) and selected organic reagents. Such reactions are carried out neat or in the presence of polar non-reactive solvents, such as ethyl acetate, at moderate temperatures, such as 50 to 130°C.

Suitable $R_f$ precursors for this purpose include the following representative compounds

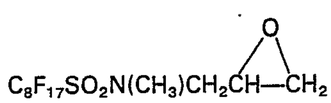

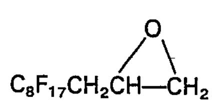

$C_8F_{17}SO_2N(CH_3)C_2H_4OCOCH{=}CH_2$

$C_8F_{17}C_2H_4OCOCH{=}CH_2$

$C_8F_{17}SO_2N(C_2H_5)C_2H_4OH$

$C_8F_{17}SO_2NHC_2H_4NH_2$

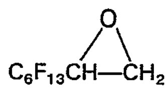

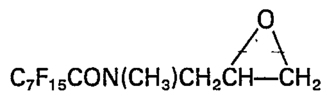

$C_7F_{15}CON(CH_3)C_2H_4OCOCH{=}CH_2$

$C_8F_{17}C_2H_4SC_2H_4OCOCH{=}CH_2$

$(CF_3)_2CF(CF_2)_8C_2H_4SCOCH{=}CH_2$

$C_8F_{17}CH_2NCO$

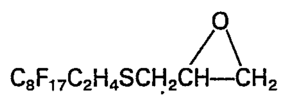

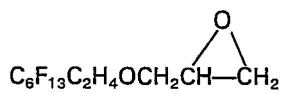

The organic reagents which are reacted with the $R_f$ precursors to prepare the fluorochemical treating agents of this invention include the following representative amines

$H_2N(CH_2)_6NH_2$

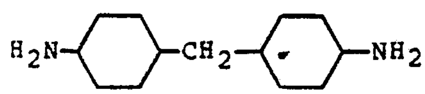

$H_2NCH_2C_6H_4CH_2NH_2$

$HOCH_2CH_2N(CH_3)CH_2CH_2OH$

$C_{18}H_{37}N(CH_3)CH_2CH_2OH$

$C_{17}H_{35}CONHC_3H_6NH_2$

$C_8H_{17}SO_2NHC_2H_4NH_2$

$H_2NNHCO(CH_2)_8CONHNH_2$

$C_7H_{15}CONHNH_2$

$H_2NCH_2CH_2NHCH_2CH_2NH_2$

the following representative electrophilic cyanoolefins

$CH_2{=}CHCN$

and the following representative polyisocyanates

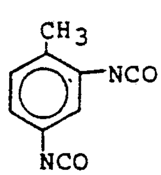

$OCN(CH_2)_6NCO$

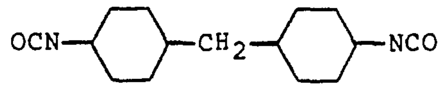

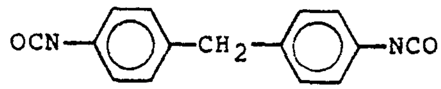

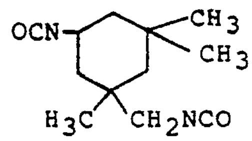

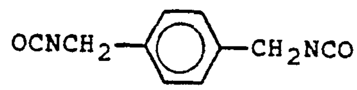

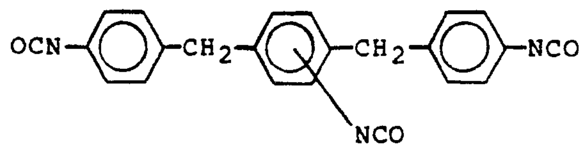

and the following representative aliphatic and aromatic epoxy compounds

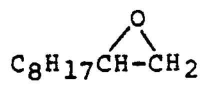

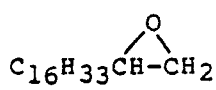

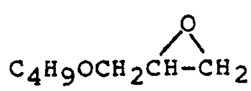

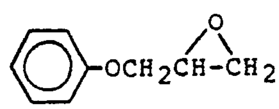

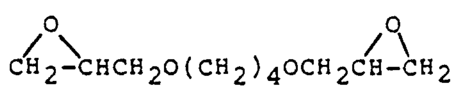

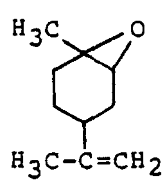

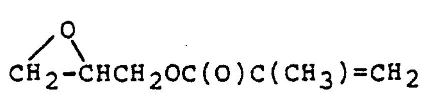

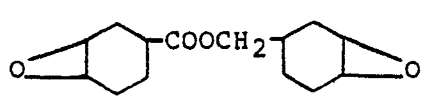

The fluorochemical amine compounds used in this invention generally can be prepared by reacting the fluoroaliphatic precursor, amine, and electrophilic cyanoolefin reactants together in appropriate molar ratios. Alternatively, such compounds can be prepared by reacting the amine and olefin together to form an adduct and then reacting the latter with the fluoroaliphatic precursor, or such compounds can be prepared by reacting the fluoroaliphatic radical precursor with the amine and then reacting the resulting adduct with the olefin. The water dispersible cationic salts of the fluorochemical amine product can be prepared by neutralizing the latter with an acid (preferably a carboxylic acid, e.g. glycolic acid) to form a salt or by reaction with an alkylating agent (such as an alkyl halide or sulfate) to form a quaternary salt. These types of reactions are generally known (see, for example, the description of a Michael reaction in "Organic Functional Group Preparations" by S. R. Sandler and W. Karo, Academic Press, N.Y. 1968, p. 334).

Representative reaction schemes for the preparation of the fluorochemical amine compounds of this invention are as follows:

Scheme I

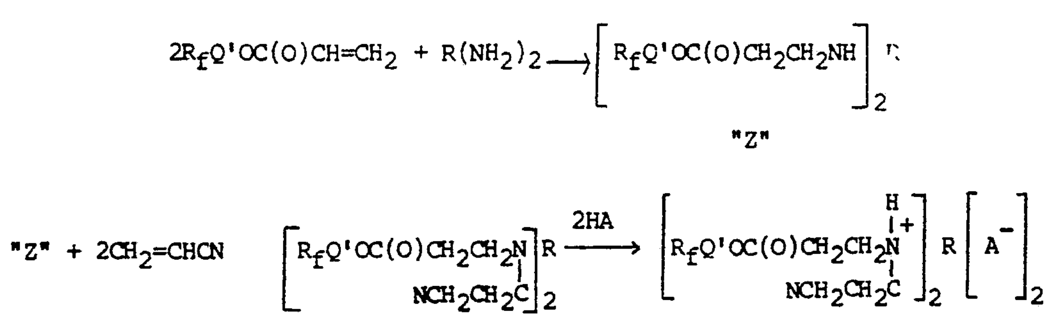


Scheme II

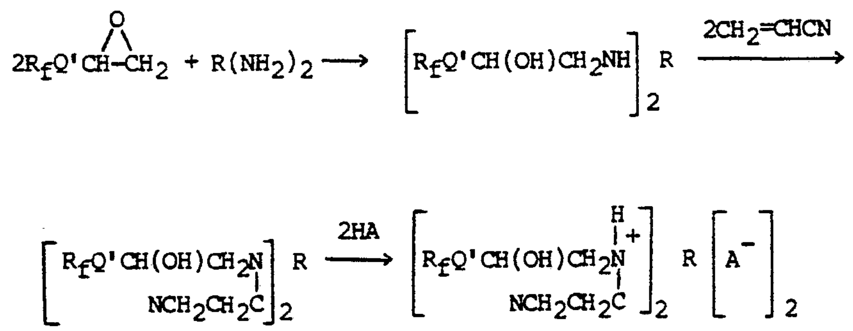


In general, the fibrous substrates are treated by contacting the surface thereof with the fluorochemical compounds of this invention. The particular mode of application of the fluorochemical compound will depend upon its particular nature as well as that of the particular substrate. The fluorochemical salt can be applied as an aqueous dispersion (or emulsion). Generally the concentration of the fluorochemical amine salt in such media will vary, as described below.

Broadly, the fibrous substrates which can be treated in accordance with this invention are textiles, paper, paperboard, leather, and the like. The textiles include those made from natural fibers, such as cotton and wool, and those made from synthetic fibers, such as nylon, acetate, rayon, acrylic, and polyester fibers. The fibers (or yarn) can be treated as such or in an aggregated form, e.g. skein or roving, with the fluorochemical compound, or the fabricated textile, e.g., articles such as carpet and woven fabrics, can be treated with the fluorochemical compound. The treatment can be carried out by applying the fluorochemicals by known techniques customarily used in applying fluorochemicals to fibrous substrates. For example, the treatment can be by immersing the fibrous substrates in a bath containing the fluorochemical, padding the substrate or spraying the same with the fluorochemical, or by foam, kiss-roll, or metering applications, e.g. spin finishing, and then drying the treated substrates. If desired, the fluorochemical can be co-applied with adjuvants, e.g. antistatic agents or neat oil (fiber lubricant).

A particularly effective method for treating textiles, e.g. carpet, with the fluorochemical compound is to carry out the treatment in conjunction with the conventional dyeing of the textiles (see, for example, the series of dyeing primer articles appearing in "Textile Chemist and Colorist," Vol. 12, Jan.—Dec., 1980). The fluorochemical can be included in the carpet dyebath, in a scour rinse prior to dyeing, or in a water rinse after dyeing. By carrying out the fluorochemical treatment in conjunction with the dyeing of the carpet, economies in manufacturing oil and water repellent carpet are realized. Where the treatment of carpet is carried out in conjunction with its dyeing, e.g. beck dyeing, by adding the fluorochemical to the dyebath,

the fluorochemical amine salt is self-emulsifiable and substantially completely exhausted from the dyebath onto the carpet fibers.

The dyebath can contain the usual adjuvants, such as buffering agents, leveling agents, pH adjusting chemicals, defoamers, softeners, sequestering agents, lubricants, etc., as well as the dyes, so long as they are compatible with the fluorochemical, which can be determined by simple laboratory screening tests. Non-ionic leveling agents are preferred for compatibility with the fluorochemical. Generally, acid, disperse, and basic (or cationic) dyes will be compatible with the fluorochemical salts of this invention.

In general, the fluorochemicals of this invention are applied at 50 to 130°C., preferably 70 to 100°C., for 10 to 60 minutes, as a solution or dispersion, e.g., in the aqueous dyebath, the concentration of the fluorochemical varying and being dependent on the mode of application. For application dependent on substantial exhaustion of the fluorochemical from the treating medium, the concentration will generally be 0.01 to 0.001 weight percent. For applications not involving exhaustion, e.g., padding, spraying, etc., higher concentrations will be needed. The amount of fluorochemical deposited on the treated substrate irrespective of the particular mode of application will be, functionally speaking, sufficient to impart the desired degree of oil and water repellency, and generally this amount will be 0.02 to 3, preferably 0.06 to 0.16, weight percent, or expressed in terms of fluorine content, 0.01 to 1.5, preferably 0.03 to 0.08, weight percent fluorine.

Following application of the fluorochemical, the treated substrate will generally be dried by heating the substrate to remove volatile material, usually and primarily water. Surprisingly, in carrying out this drying, the temperature necessary to obtain the desired degree of oil and water repellency for many of the fluorochemicals of this invention will be relatively lower than that usually required in conventional industry practice. For example, such properties can be achieved by drying treated carpet at 70°C. in accordance with this invention, whereas a drying (curing) temperature of 100—130°C. is common practice in treating carpet with fluorochemical.

Objects and advantages of this invention are illustrated in the following examples, the first series of examples describing the preparation of various fluorochemical compounds of this invention, the second series of examples describing their evaluation in the treatment of carpet during the dyeing thereof, and the balance of the examples describing the use of the fluorochemicals of this invention in the treatment of various other substrates.

Example 1

In a 3-neck, 250 ml borosilicate glass flask, fitted with condenser, thermometer, stirrer, and electric heating mantle, were placed 58.3 g (0.1 mole) of N-methyl-N-(1,2 epoxypropyl)perfluorooctanesulfonamide, 5.8 g (0.05 mole) hexamethylene diamine, and 5.3 g (0.1 mole) of acrylonitrile. The resulting reaction mixture was heated to 80°C. and stirred for about four hours, the resulting viscous product solidifying on cooling the reaction mixture to room temperature. This product, a fluorochemical amine adduct, having the formula

$$\left[C_8F_{17}SO_2N(CH_3)CH_2CH(OH)CH_2\underset{CH_2CH_2CN}{\underset{|}{N}}\!\!-\!(CH_2)_3\!-\right]_2 \qquad \underline{1A}$$

was mixed with 12 g. of 80 percent aqueous (0.1 mole) glycolic acid. The resulting mixture was heated to 80°C., mixed vigorously with 280 ml deionized water to yield a stable 20 percent aqueous dispersion of cationic fluorochemical amine salt compound having the formula:

$$\left[C_8F_{17}SO_2N(CH_3)CH_2CH(OH)CH_2\overset{H}{\underset{CH_2CH_2CN}{N^+}}\!\!-\!(CH_2)_3\!-\right]_2\left[HOCH_2COO^-\right]_2 \qquad \underline{1}$$

Examples 2—5

Other salts of the above-described adduct *1A* of Example 1 were prepared in a similar manner except that the glycolic acid was replaced with various other acids, or with diethyl sulfate alkylating agent. Stable aqueous dispersions of the resulting cationic fluorochemicals were prepared as described above. The formulas for these salts are:

$$\left[R_fQ\overset{H}{\underset{CH_2CH_2CN}{N^+}}\!\!-\!(CH_2)_3\!-\right]_2\left[\begin{array}{c}CH_2COO^-\\ |\\ HOCCOOH\\ |\\ CH_2COO^-\end{array}\right] \qquad \underline{2}$$

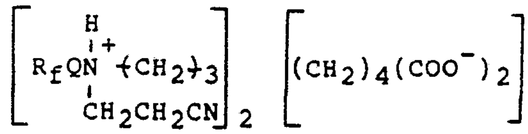 3

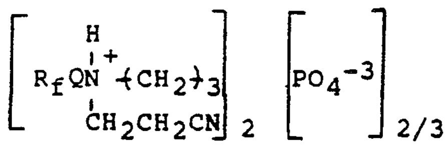 4

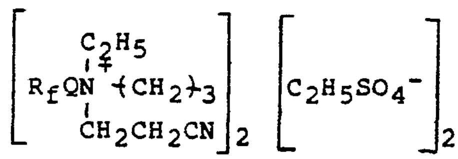 5

where $R_f$ is $C_8F_{17}$— and Q is —$SO_2N(CH_3)CH_2CH(OH)CH_2$—.

Example 6

Another fluorochemical amine salt compound of the formula

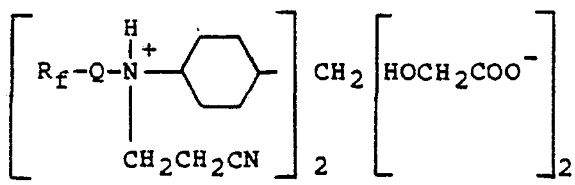 6

where $R_f$ and Q are as in formulas 2—5, was prepared in the manner similar to that of Example 1 except that in place of hexamethylene diamine there was used methylenebis(cyclohexylamine).

Example 7

Another fluorochemical amine salt compound was prepared in a manner similar to Example 1 by reacting 1,2-epoxy-3-perfluorooctyl-1,1,2,3,3-pentahydropropane (in ethyl acetate solvent) with hexamethylene diamine, and then reacting the resulting adduct with acrylonitrile.

The resulting product of the formula

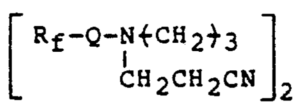 7A

was neutralized with glycolic acid to form a salt of the formula

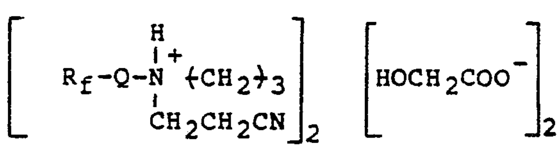 7

where $R_f$ is $C_8F_{17}$— and Q is —$CH_2CH(OH)CH_2$.

Examples 8—10

Three other fluorochemical amine salt compounds were prepared by reacting hexamethylene diamine or methylenebis(cyclohexylamine) with N-methylperfluorooctanesulfoamidoethyl acrylate, then reacting the resulting adducts with acrylonitrile, and then forming the salt, following the procedure of Example 1.

The formulas for these three salts are

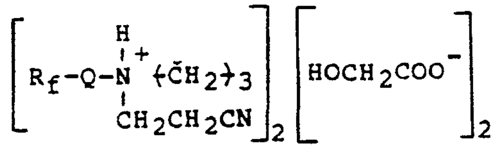

8

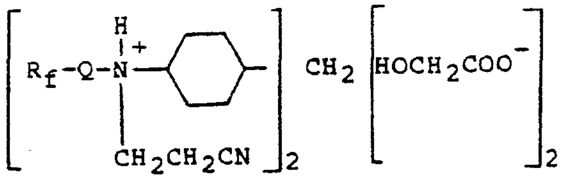

9

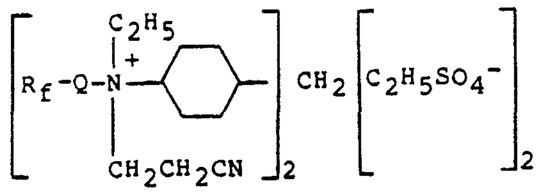

10

where $R_f$ is $C_8F_{17}$— and Q is —$SO_2N(CH_3)C_2H_4OCOC_2H_4$—.

Example 11

Another fluorochemical amine salt was prepared in a manner similar to Example 1 by reacting N-methylperfluorooctanesulfonamidoethyl acrylate with perfluorooctanesulfonamidoethyl amine in ethyl acetate solvent, then reacting the resulting adduct with acrylonitrile, the resulting product then being neutralized with glycolic acid to form the corresponding salt, the formula of which is

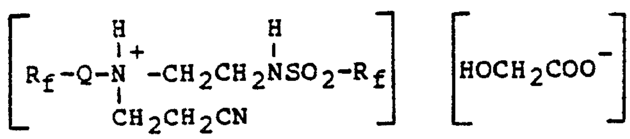

11

where $R_f$ and Q are as in formula *8*.

Example 12

In this example, a number of the fluorochemicals prepared as described above were used in modifying the surface properties of carpet samples during the dyeing thereof in a laboratory dyeing apparatus. The samples of carpet were that of a scoured, greige nylon 6,6 carpet having a pile weight of 900 g/m², 5 stitches/cm, and a pile height of 0.635 cm, each sample weighing 25 g.

The laboratory dyeing apparatus was that sold as "AHIBA Turbomat™ TM6B" (whose construction and operation is described in bulletin A—102—B—DE—1) of Ahiba AG.

The dye bath composition for each of the evaluation runs made was as follows:

TABLE 1

| Ingredient | Amt., ml |
|---|---|
| 1. Deionized water | 750 |
| 2. $Na_2SO_4$ (10% aqueous solution) | 2.5 |
| 3. Dye* (1.9% aqueous solution) | 2.5 |
| 4. Non-ionic leveling agent (Levegal™ ANP) (10% aqueous dispersion) | 2.5 |
| 5. Fluorochemical amine salt compound (10% aqueous dispersion) | 2.5 |
| 6. Acetic acid (1% aqueous solution) | 5 |

* A mixture of 3.5 g Nylosan™ Red C—BNL, 4.4 g Tectilon™ Blue 4R—KWL, and 11.0 g Tectilon™ Yellow 913 D.

In each run, the carpet sample was wrapped around the perforated metal sample holder, secured with rubber bands and placed in a dyeing beaker, and the beaker placed in the bath casing. Then the above-described dye bath composition (except for the acetic acid) were added in the order listed above and the resulting bath contents heated to 96°C and held at this temperature for 45 minutes, during which time the bath was circulated through the carpet sample by the magnetically driven pump disposed at the lower end of the dyeing beaker. The acetic acid ingredient was added in two increments during the 45 minute period, the first 2.5 ml after 15 minutes and the second 2.5 ml after 30 minutes. The sample holder, with carpet sample still attached thereto was removed from the dye bath after it cooled to about 60°C and placed in a cooled water rinse bath. The sample was then removed from the holder and centrifuged to remove most of the water. The carpet sample was then dried in air at 70°C for 10 min. The dried carpet samples were then evaluated for water and oil repellency.

The water repellency test is one which often is used for this purpose. The aqueous stain or water repellency of treated samples is measured using a water/isopropyl alcohol test, and is expressed in terms of a water repellency rating of the treated carpet or fabric. Treated carpets which are penetrated by or resistant only to a 100 percent water/0 percent isopropyl alcohol mixture, the least penetrating of the test mixtures, are given a rating of 100/0, whereas treated fabrics resistant to a 0 percent water/100 percent isopropyl alcohol mixture, the most penetrating of the test mixtures, are given a rating of 0/100. Other intermediate values are determined by use of other water/isopropyl alcohol mixtures, in which the percentage amounts of water and isopropyl alcohol are each multiples of 10. The water repellency rating corresponds to the most penetrating mixture which does not penetrate or wet the fabric after 15 seconds contact. In general a water repellency rating of 90/10 or better is desirable.

The oil repellency test is also one which is often used for this purpose. The oil repellency of treated carpet and textile samples is measured by AATCC Standard Test 118—1978, which test is based on the resistance of treated fabric to penetration by oils of varying surface tensions. Treated fabrics resistant only to Nujol™, a brand of mineral oil, and the least penetrating of the test oils, are given a rating of 1, whereas treated fabrics resistant to heptane, the most penetrating of the test oils, are given a value of 8. Other intermediate values are determined by use of other pure oils or mixtures of oils. The rated oil repellency corresponds to the most penetrating oil (or mixture of oils) which does not penetrate or wet the fabric after 30 seconds contact. Higher numbers indicate better oil repellency. In general, an oil repellency of 2 or greater is desirable.

The following table summarizes the results obtained by treating carpet samples with various fluorochemicals during the dyeing of the carpet samples, as described above.

TABLE 2

| Formula of Fluorochemical Used | Properties of Treated Carpet Samples | |
|---|---|---|
| | Oil Repellency | Water Repellency |
| 1 | 5 | 70/30 |
| 2 | 4 | 80/20 |
| 3 | 5 | 70/30 |
| 4 | 5 | 70/30 |
| 5 | 5 | 70/30 |
| 6 | 3 | 80/20 |
| 7 | 5 | 80/20 |
| 8 | 0 | 90/10 |
| 9 | 3 | 90/10 |
| 10 | 1 | 90/10 |
| 11 | 3 | 90/10 |

As shown in Table 2, most of the fluorochemical compounds of this invention imparted desirable repellency properties to the carpet samples.

Example 13

In a number of runs, the procedure of Example 12 was followed, except that instead of adding fluorochemical to the dye bath, the fluorochemical (formula *1*) was added to the water rinse bath to provide it in the amount of 1800 ppm, together with a salt added to the rinse bath to aid exhaustion of the fluorochemical. The water rinse bath had a pH of about 4 to 5. A summary of the runs is shown in the following Table.

TABLE 3

| Run | Salt Exhaustion Aid | Oil Repellency | Water Repellency |
|---|---|---|---|
| 1 | $NaSO_4$ | 5 | 70/30 |
| 2 | $MgCl_2$ | 2 | 80/20 |
| 3 | $(NH_4)_2SO_4$ | 5 | 70/30 |
| 4 | $(Al)_2(SO_4)_3$ | 4 | 70/30 |
| 5 | Sodium citrate | 5 | 70/30 |
| 6 | $CaCl_2$ | 2 | 80/20 |
| 7 | $NH_4Cl$ | 2 | 80/20 |
| 8 | KCl | 2 | 70/30 |
| 9 | none | 0 | 80/20 |

Example 14

Fluorochemical treating agents of Examples 1A, 1, 7A, 7, and 8, whose preparations are described herein above, were used to treat carpet samples, using for this purpose a laboratory dyeing machine sold under the trademark Launder-Ometer, Model LEF, as described in Bulletin No. 1295 B, of the Atlas Electric Devices Company. The carpet used was a nylon 6,6 which was the same as in the previously described examples. The bath used in applying the fluorochemical had the following composition.

TABLE 4

| Treating Bath Ingredient | Amt., ml |
|---|---|
| 1. Deionized water | 800 |
| 2. $(NH_4)_2SO_4$ buffer (5% aqueous) | 4 |
| 3. Fluorochemical (20% aqueous dispersion) | 0.3 |
| 4. Non-ionic leveling agent (Tridye™, 10% aqueous solution) | 2 |

In each run, the carpet sample was placed in a steel specimen container, which was then mounted on a rotor adapted to be rotated in a constant temperature water bath. The loaded specimen container is heated to 96°C and rotated for 45 minutes, cooled to about 30°C, and the treated carpet samples are then removed from the container, rinsed, centrifuged to remove excess water, and dried for 30 minutes at 70°C. The oil repellency and water repellency of the dried samples were then tested by the procedure described herein above. In addition, the amount of fluorochemical deposited on the carpet samples was found by determining the amount of elemental fluorine in order to thereby find the degree of exhaustion or deposition of the fluorochemical from the treating bath. Results are summarized in the following table.

TABLE 5

| Run | Formula of Fluorochemical Used | Oil Repellency | Water Repellency | Amt. Fluorine Deposited, ppm | Degree of Fluorochemical Exhausted, % (approx.) |
|---|---|---|---|---|---|
| 1 | *1A* | 0 | 100/0 | 360 | 60 |
| 2 | *1* | 5 | 70/30 | 750 | 100 |
| 3 | *7A* | 0 | 80/20 | 540 | 90 |
| 4 | *7* | 2 | 80/20 | 630 | 100 |
| 5 | *8* | 5 | 70/30 | 450 | 75 |

The above table shows in Runs 1—4 that when the fluorochemical is in the form of its salt form formulas *1* and *7*, the oil repellency of the carpet treated therewith is significantly greater, and the water repellency of the treated carpet is also better or equivalent, to those results obtained when the fluorochemical was in its free amine form, formulas *1A* and *7A*.

The above data also show that approximately 100 percent of the fluorochemical in the treating bath is exhausted in the case where such adduct is used, viz., the fluorochemicals of formulas *1* and *7*.

Example 15

An aqueous dispersion of 0.4 wt.% of the fluorochemical treating agent of formula *1* was applied as a primary spin finish (together with a coconut-oil based lubricant) to freshly extruded, undrawn yarn of nylon 6 fibers. The yarn was made up of 118 filaments of 18 denier and the fluorochemical was applied using a commercial spin finish applicator. The thus treated yarn was continuously drawn and texturized, and then made into level-loop carpet (945 g/m$^2$), heat set at 196°C for one minute, acid dyed, dried, and then evaluated for oil and water repellency. The water repellency was 70/30 and the oil repellency was 4, using the test procedure herein above described. The treated sample was also analyzed to determine the amount of fluorochemical deposited and it was found that it contained 320 ppm fluorine, since the corresponding amount of fluorine deposited on the fiber before dyeing was 470 ppm, the amount of fluorochemical retained after dyeing was about 70 percent. The soil resistance of the carpet was also evaluated by a "Walk-On Test" often used in the carpet industry, viz., AATCC Test Method 122—1976. This is a comparative test in which a treated and untreated (control) sample are placed side-by-side in a heavily travelled industrial area for an exposure of about 7,000 steps. The samples are rotated periodically to insure uniform exposure and are vacuumed every 24 hours during the test and before visual evaluation. Using this test, the treated carpet was found to have a significant improvement in soil resistance when compared to the untreated sample.

Example 16

A loosely knitted fabric of carrierless polyester carpet yarn (12 denier per filament), was treated with an aqueous dispersion of 0.18 wt.% of the fluorochemical of formula *1* by a padding operation. The treated fabric was dried for 15 minutes at 160°C, disperse dyed, using the apparatus of Example 14, and dried. The treated fabric was found to have an oil repellency of 5 and a water repellency value of 80/20. The dyed fabric was found to have 390 ppm fluorine (as compared to 614 ppm fluorine before dyeing), thus 64 percent of the fluorochemical treating agent deposited was retained after dyeing.

Example 17

Nylon taffeta apparel fabric (light weight, single ply, uncoated) was sprayed with an aqueous dispersion of 0.42 wt.% fluorochemical treating agent of formula *1*. Wet pick up was at the 25 percent level, thus depositing 0.1 wt.% fluorochemical solid on the fabric. The treated fabric was then dried for 10 minutes at 150°C, and the oil repellency was determined by the test herein above described. The water spray rating of the treated fabric was also evaluated.

The water spray rating is measured by Standard Test Number 22, published in the 1977 Technical Manual and Yearbook of the American Association of Textile Chemists and Colorists (AATCC). The spray rating is measured using a 0 to 100 scale where 100 is the highest possible rating. In general, a spray rating of 70 or greater is desirable, particularly for outerwear fabrics.

In a similar top spray application, the fluorochemical treating agent was applied to an apparel fabric made of woven polyester (style Seville™/Suraline™), and an apparel fabric made of polyester double knit (style 1418), the amounts deposited on these fabrics being 0.3 wt.% and 0.4 wt.%, respectively. Results are shown in the following table.

TABLE 6

| Fabric | Oil Repellency | Water Spray Rating |
|---|---|---|
| Nylon (taffetta) | 6 | 50 |
| Polyester (woven) | 5 | 70 |
| Polyester (double knit) | 6 | 70 |

Example 18

A sample of chrome-tanned leather was treated with an aqueous dispersion of 0.67 wt.% of the fluorochemical of formula *1* in a drumming operation. The amount of fluorochemical deposited on the leather was 2 wt.%. The treated leather was dried in air. The grain and suede sides of the treated leather were found to have oil repellency values of 4 and 6 respectively and the water repellency values for the grain and suede sides were found to be 80/20 on both sides. (Untreated chrome-tanned leather has essentially no oil or water repellency.)

Similarly, a sample of chrome-tanned, dyed, fat liquored leather was treated with the fluorochemical of formula *1* and was found to have oil repellency values of 2 and 5 on the grain and suede side, respectively, and a water repellency value of 80/20 and 70/30 for the grain and suede sides respectively.

Example 19

The fluorochemical of formula *1* was applied as an aqueous dispersion to a sheet of water-leaf paper using a laboratory press yielding 145 percent wet pick up. The results of three such runs are summarized in the following table.

TABLE 7

| Run | Formula of Fluorochemical Used | Conc. of Fluorochemical in Bath, wt.% | Amount of Fluorochemical on Paper wt.% | Oil Repellency* | Water Repellency** |
|---|---|---|---|---|---|
| 1 | *1* | 0.07 | 0.1 | 7 | No water repellency |
| 2 | *1* | 0.14 | 0.2 | 7 | No water repellency |
| 3 | *1* | 0.21 | 0.3 | 8 | 75 |

* This was determined by the "Kit Test" described as TAPPI Useful Method 557; the higher the value, the better the oil repellency.

** This was determined by the "Cobb Test" described as TAPPI—T441—os—77; the lower the value, the better the water repellency.

The data in the above table show that for some fluorochemicals of this invention, large amounts of them may have to be deposited on the paper to obtain desired water repellency. However, such flurochemical can be used in an amount sufficient to obtain the desired degree of oil repellency and they can be used in conjunction with hydrocarbon treating agents, e.g. ketene dimers, commonly used to impart water repellency to paper.

Various modifications and alterations of this invention will be apparent to those skilled in the art without departing from the scope and spirit of this invention.

**Claims**

1. A process for treating fibrous substrates to impart oil and water repellency to the surface thereof, by means of contacting said substrate with a fluorochemical amine salt compound containing one or two fluoroaliphatic radicals and one or two tertiary amine salt groups or quaternary amine salt groups, characterized in that said compounds contain as N-substituents of the tertiary amine salt groups or the quaternary amine salt groups, electron-withdrawing cyanoalkyl moieties.

2. The process of claim 1 further characterized by the feature that said amine salt compound has the formula

$$\text{I} \qquad \left[ R_f-Q-\underset{\substack{| \\ R^3}}{\overset{\substack{R^1 \\ |}}{N}}-R^2 \right]_y (R^4)_p \left[ A \right]_z$$

where

$R_f$ is a fluoroaliphatic radical,

Q is an organic linking moiety,

N is a tertiary amine nitrogen or a quaternary amine nitrogen,

$R^1$ is a hydrogen or an alkyl group,

$R^2$ is a monovalent group selected from the class of $R_fQ$—, alkyl and aryl groups, and combinations of such groups, which groups can contain a hetero atom, RCONH— (where R is alkyl or aryl), and

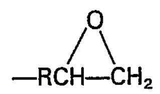

(where R is alkylene), or $R^2$ is a divalent group selected from the class of alkylene and arylene groups, combinations thereof, and —RCONH— (where R is alkylene or arylene),

$R^3$ is an electron-withdrawing cyanoalkyl moiety,

$R^4$ is an alkylene or arylene group,

A is an anion derived from a protonic acid or an alkylating agent,

y is 1 or 2,

p is 0 or 1,

z is 1 or 2,

with the provisos that

when y is 1, then p is 0 and z is 1,

when y is 2, then $R^2$ is said divalent group.

3. The process of claim 1, further characterized by the feature that said substrate is textile fiber, paper, or leather.

4. The process of claim 1, further characterized by the feature that said substrate is carpet.

5. The process of claim 1, further characterized by the feature that said contacting is carried out by immersing said substrate in an aqueous bath containing said amine salt compound.

6. The process of claim 5, further characterized by the feature that said bath is a dyebath containing a dye for said substrate.

7. The process of claim 1, further characterized by the feature that said amine salt compound has the formula

$$\left[ C_8F_{17}SO_2N(CH_3)CH_2CH(OH)CH_2\underset{\substack{| \\ CH_2CH_2CN}}{\overset{\substack{H \\ |}}{N^+}}\!\!-\!(CH_2)_3 \right]_2 \left[ HOCH_2COO^- \right]_2$$

8. A fibrous substrate having deposited on the surface thereof fluorochemical amine salt compound containing one or two fluoroaliphatic radicals and one or two tertiary amine salt groups or quaternary amine salt groups, characterized in that said compounds contain as N-substituents of the tertiary amine salt groups or the quaternary amine salt groups, electron-withdrawing cyanoalkyl moieties.

9. Fluorochemical amine salt compounds containing one or two fluoroaliphatic radicals and one or two tertiary amine salt groups or quaternary amine salt groups, characterized in that said compounds contain as N-substituents of the tertiary amine salt groups or the quaternary amine salt groups, electron-withdrawing cyanoalkyl moieties.

10. Compounds of claim 9 further characterized by the formula

$$\text{I} \qquad \left[ R_f-Q-\underset{\substack{| \\ R^3}}{\overset{\substack{R^1 \\ |}}{N}}-R^2 \right]_y (R^4)_p \left[ A \right]_z$$

where

$R_f$ is a fluoroaliphatic radical,

Q is an organic linking moiety,

N is a tertiary amine nitrogen or a quaternary amine nitrogen,

$R^1$ is a hydrogen or an alkyl group,

$R^2$ is a monovalent group selected from the class of $R_fQ$—, alkyl and aryl groups, and combinations of such groups, which groups can contain a hetero atom, RCONH— (where R is alkyl or aryl), and

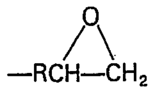

(where R is alkylene), or $R^2$ is a divalent group selected from the class of alkylene and arylene groups, combinations thereof, and —RCONH— (where R is alkylene or arylene),

$R^3$ is an electron-withdrawing cyanoalkyl moiety,

$R^4$ is an alkylene or arylene group,

A is an anion derived from a protonic acid or an alkylating agent,

y is 1 or 2,

p is 0 or 1,

z is 1 or 2,

with the provisos that

when y is 1, then p is 0 and z is 0 or 1,

when y is 2, then $R^2$ is said divalent group.

11. A compound of claim 9 further characterized by the formula

$$\left[C_8F_{17}SO_2N(CH_3)CH_2CH(OH)CH_2\overset{H}{\underset{CH_2CH_2CN}{N^+}}\text{-}(CH_2)_3\right]_2\left[HOCH_2COO^-\right]_2$$

12. A compound of claim 9 further characterized by the formula

$$\left[C_8F_{17}SO_2N(CH_3)C_2H_4OCOC_2H_4\overset{C_2H_5}{\underset{CH_2CH_2CN}{N^+}}\text{-}(CH_2)_3\right]_2\left[C_2H_5SO_4^-\right]_2$$

**Patentansprüche**

1. Verfahren zum Behandeln von Fasermaterialsubstraten zwecks Erzielung einer öl- und wasserabweisenden Oberfläche, indem das Substrat mit einer fluorchemischen Aminsalzverbindung in Berührung gebracht wird, die ein oder zwei fluoraliphatische Radikale und eine oder zwei tertiäre oder quaternäre Aminsalzgruppen enthält, dadurch gekennzeichnet, daß die genannten Verbindungen als N-Substituenten der tertiären oder quaternähren Aminsalzgruppen elektronenabziehende Cyanalkylanteile enthalten.

2. Verfahren nach Anspruch 1, dadurch gekennzeichnet, daß die Aminsalzverbindung die Formel

$$\text{I} \qquad \left[R_f\text{-}Q\text{-}\overset{R^1}{\underset{R^3}{N}}\text{-}R^2\right]_y (R^4)_p \left[A\right]_z$$

hat, in der

$R_f$ ein fluoraliphatisches Radikal,

Q eine organische Verkettung,

N ein Stickstoffatom eines tertiären oder quaternären Amins und

$R^1$ Wasserstoff oder eine Alkylgruppe ist,

$R^2$ eine einwertige Gruppe ist, die aus der Klasse ausgewählt ist, die aus den $R_fQ$-Gruppen, den Alkyl- und Arylgruppen und Kombinationen dieser Gruppen ausgewählt ist, wobei diese Gruppen ein Heteroatom, RCONH— (wobei R Alkyl oder Aryl ist) und

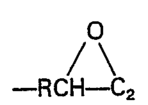

(wobei R Alkylen ist) enthalten können, oder $R^2$ eine zweiwertige Gruppe ist, die aus der Klasse ausgewählt ist, die aus den Alkylen- und Arylengruppen, deren Kombinationen und —RCONH— (wobei R Alkylen oder Arylen ist) besteht,

$R^3$ ein elektronenabziehender Cyanalkylanteil,
$R^4$ eine Alkylen- oder Arylengruppe und
A ein von einer Brönstedt-Serie oder einem Alkylierungsmittel abgeleitetes Anion,
$y = 1$ oder 2
$p = 0$ oder 1 und
$z = 1$ oder 2
ist, und
wenn $y = 1$ ist, $p = 0$ und $z = 1$ ist und
wenn $y = 2$ ist, $R^2$ die genannte zweiwertige Gruppe ist.

3. Verfahren nach Anspruch 1, dadurch gekennzeichnet, daß das Substrat aus Textilfasern, Papier oder Leder besteht.

4. Verfahren nach Anspruch 1, dadurch gekennzeichnet, daß das Substrat ein Teppich ist.

5. Verfahren nach Anspruch 1, dadurch gekennzeichnet, daß zum Inberührungbringen das Substrat in ein die Aminsalzverbindung enthaltendes, wäßriges Bad getaucht wird.

6. Verfahren nach Anspruch 5, dadurch gekennzeichnet, daß das Bad eine Farbflotte ist, die einen Farbstoff für das Substrat enthält.

7. Verfahren nach Anspruch 1, dadurch gekennzeichnet, daß die Aminsalzverbindung die Formel

$$\left[C_8F_{17}SO_2N(CH_3)CH_2CH(OH)CH_2\overset{H}{\underset{CH_2CH_2CN}{N^+}}\!\!-\!(CH_2)_3-\right]_2\left[HOCH_2COO^-\right]_2$$

hat.

8. Fasermaterialsubstrat, auf dessen Oberfläche fluorchemische Aminsalzverbindungen abgelagert sind, die ein oder zwei fluoraliphatische Radikale und eine oder zwei tertiäre oder quaternäre Aminsalzgruppen enthält, dadurch gekennzeichnet, daß die genannten Verbindungen als N-Substituenten der tertiären oder quaternären Aminsalzgruppen elektronenabziehende Cyanalkylanteile enthalten.

9. Fluoraliphatische Aminsalzverbindungen, die ein oder zwei fluoralliphatische Radikale und eine oder zwei tertiäre oder quaternäre Aminsalzgruppen enthalten, dadurch gekennzeichnet, daß die genannten Verbindungen als N-Substituenten der tertiären oder quaternären Aminsalzgruppen elektronenabziehende Cyanalkylanteile enthalten.

10. Verbindungen nach Anspruch 9, gekennzeichnet durch die Formel

I

$$\left[R_f-Q-\overset{R^1}{\underset{R^3}{N}}-R^2\right]_y (R^4)_p \left[A\right]_z$$

in der
$R_f$ ein fluoraliphatisches Radikal,
Q eine organische Verkettung,
N ein Stickstoffatom eines tertiären oder quaternären Amins und
$R^1$ Wasserstoff oder eine Alkylgruppe ist,
$R^2$ eine einwertige Gruppe ist, die aus der Klasse ausgewählt ist, die aus den $R_fQ$-Gruppen, den Alkyl- und Arylgruppen und Kombinationen dieser Gruppen ausgewählt ist, wobei diese Gruppen ein Heteroatom, RCONH (wobei R Alkyl oder Aryl ist) und

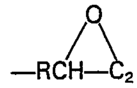

(wobei R Alkylen ist) enthalten können, oder $R^2$ eine zweiwertige Gruppe ist, die aus der Klasse ausgewählt ist, die aus den Alkylen- und Arylengruppen, deren Kombinationen und —RCONH— (wobei R Alkylen oder Arylen ist) besteht,
$R^3$ ein elektronenabziehender Cyanalkylanteil
$R^4$ eine Alkylen- oder Arylengruppe und
A ein von einer Brönstedt-Serie oder einem Alkylierungsmittel abgeleitetes Anion,
$y = 1$ oder 2
$p = 0$ oder 1 und
$z = 1$ oder 2
ist, und
wenn $y = 1$ ist, $p = 0$ und $z = 1$ ist und
wenn $y = 2$ ist, $R^2$ die genannte zweiwertige Gruppe ist.

11. Verbindung nach Anspruch 9, gekennzeichnet durch die Formel

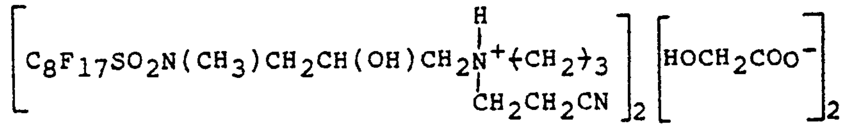

12. Verbindung nach Anspruch 9, gekennzeichnet durch die Formel

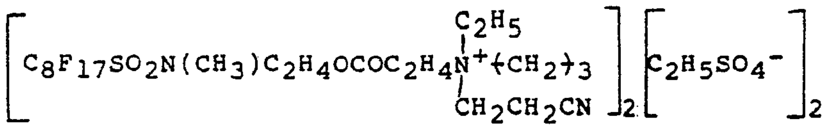

**Revendications**

1. Procédé de traitement de substrats fibreux pour conférer une oléophobie et une hydrophobie à leur surface par contact dudit substrat avec un composé chimique qui est un sel d'amine fluoré contenant 1 ou 2 radicaux fluoroaliphatiques et 1 ou 2 groupes sel d'amine tertiaire ou groupes sel d'amine quaternaire, caractérisé en ce que ledit composé contient en tant que N-substituants des groupes sel d'amine tertiaire ou des groupes sel d'amine quaternaire, des fragments cyanoalkyle attracteurs d'électrons.

2. Procédé suivant la revendication 1 caractérisé de plus par le fait que ledit sel d'amine a pour formule

I 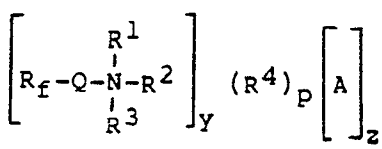

dans laquelle

$R_f$ est un radical fluoro aliphatique,

Q est un fragment de liaison organique,

N est un azote d'amine tertiaire ou un azote d'amine quaternaire,

$R^1$ est un hydrogène ou un groupe alkyle,

$R^2$ est un groupe monovalent choisi parmi l'ensemble comprenant les groupes $R_fQ$—, alkyle et aryle et les combinaisons de tels groupes, lesquels groupes peuvent contenir un hétéroatome, RCONH— (où R est alkyle ou aryle) et

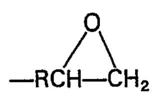

(où R est alkylène) ou $R^2$ est un groupe divalent choisi parmi l'ensemble comprenant les groupes alkylène et arylène, leurs combinaisons, et —RCONH— (où R est alkylène ou arylène),

$R^3$ est un fragment cyanoalkyle attracteur d'électrons,

$R^4$ est un groupe alkylène ou arylène,

A est un anion dérivé d'un acide protonique ou d'un agent d'alkylation,

y est 1 ou 2,

p est 0 ou 1,

z est 1 ou 2,

avec les conditions que

lorsque y est 1, p est alors 0 et z est 1, et

lorsque y est 2, $R^2$ est alors ledit groupe divalent.

3. Procédé suivant la revendication 1, caractérisé de plus par le fait que ledit substrat est fait de fibres textiles, de papier ou de cuir.

4. Procédé suivant la revendication 1, caractérisé de plus par le fait que ledit substrat est un tapis.

5. Procédé suivant la revendication 1, caractérisé de plus par le fait que ledit contact est effectué par immersion dudit substrat dans un bain aqueux contenant ledit sel d'amine.

6. Procédé suivant la revendication 5, caractérisé de plus par le fait que ledit bian est un bain de teinture contenant un colorant pour ledit substrat.

7. Procédé suivant la revendication 1, caractérisé de plus par le fait que ledit sel d'amine a pour formule

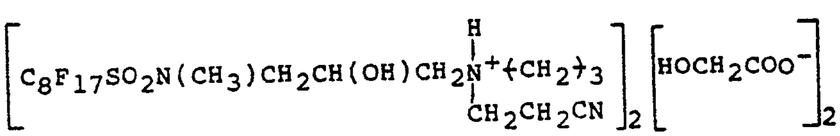

8. Substrat fibreux à la surface duquel est déposé un composé chimique qui est un sel d'amine fluoré contenant 1 ou 2 radicaux fluoroaliphatiques et 1 ou 2 groupes sel d'amine tertiaire ou groupes sel d'amine quaternaire caractérisé en ce que ledit composé contient en tant que N-substituants des groupes sel d'amine tertiaire ou des groupes sel d'amine quaternaire, des fragments cyanoalkyle attracteurs d'électrons.

9. Composés de sel d'amine fluoré contenant un ou deux radicaux fluoroaliphatiques et un ou deux groupes sel d'amine tertiaire ou groupes sel d'amine quaternaire, lesdits composés étant caractérisés en ce qu'ils contiennent, comme N-substituants des groupes sel d'amine tertiaire ou des groupes sel d'amine quaternaire, des fragments cyanoalkyle attracteurs d'électrons.

10. Composés suivant la revendication 9, caractérisés de plus par la formule

I 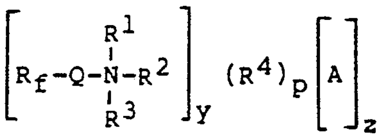

dans laquelle

$R_f$ est un radical fluoroaliphatique,

Q est un fragment de liaison organique,

N est un azote d'amine tertiaire ou un azote d'amine quaternaire,

$R^1$ est un hydrogène ou un groupe alkyle,

$R^2$ est un groupe monovalent choisi parmi l'ensemble comprenant les groupes $R_fQ$—, alkyle et aryle et les combinaisons de tels groupes, lesquels groupes peuvent contenir un hétéroatome, RCONH— (où R est alkyle ou aryle) et

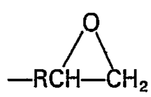

(où R est alkylène), ou $R^2$ est un groupe divalent choisi parmi l'ensemble comprenant les groupes alkylène et arylène, leurs combinaisons, et —RCONH— (où R est alkylène ou arylène),

$R^3$ est un fragment cyanoalkyle attracteur d'électrons,

$R^4$ est un groupe alkylène ou arylène,

A est un anion dérivé d'un acide protonique ou d'un agent d'alkylation,

y est 1 ou 2,

p est 0 ou 1,

z est 1 ou 2,

avec les conditions que

lorsque y est 1, p est alors 0 et z est 1, et

lorsque y est 2, $R^2$ est alors ledit groupe divalent.

11. Composé suivant la revendication 9, caractérisé de plus par la formule

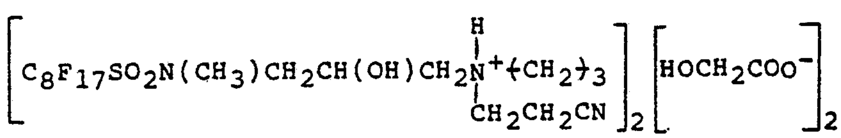

12. Composé suivant la revendication 9, caractérisé de plus par la formule